# EUROPÄISCHE PATENTSCHRIFT

(11) **EP 4 308 034 B1**
(45) Veröffentlichungstag und Bekanntmachung des Hinweises auf die Patenterteilung: **14.10.2026**
(21) Anmeldenummer: 22713906.0
(22) Anmeldetag: 10.03.2022
(51) Int. Cl.: A61B 90/98, G06K 19/077

(54) **GLASS-TAG TRANSPONDER TORSIONS-SCHUTZGEHÄUSE**
TORSION-PROTECTION HOUSING FOR GLASS TAG TRANSPONDERS
BOÎTIER DE PROTECTION CONTRE LA TORSION POUR TRANSPONDEURS D'ÉTIQUETTES EN VERRE

(30) Priorität: 17.03.2021 DE 102021106454
(43) Veröffentlichungstag der Anmeldung: 24.01.2024
(73) Patentinhaber: Aesculap AG, 78532 Tuttlingen (DE)
(72) Erfinder: PFISTER, Ralf, 78647 Trossingen (DE); LENZENHUBER, Frederick, 78532 Tuttlingen (DE); AUBER, Stephanie, 78532 Tuttlingen (DE); HÖGERLE, Roland-Alois, 78532 Tuttlingen (DE)
(74) Vertreter: Winter, Brandl - Partnerschaft mbB
(86) Internationale Anmeldenummer: PCT/EP2022/056163
(87) Internationale Veröffentlichungsnummer: WO 2022/194663

(56) Entgegenhaltungen:
- EP-A1- 2 423 855
- WO-A1-2014/045265

## Beschreibung

Die vorliegende Offenbarung betrifft ein Schutzgehäuse zum Anbringen eines darin aufnehmbaren Transponders in/an einem medizinischen Produkt, insbesondere in einem chirurgischen Instrument und / oder Medizinprodukt in einem Sterilisationskreislauf. Weiterhin betrifft die Offenbarung ein medizinisches System mit einem Schutzgehäuse und einem medizinischen Produkt.

### Hintergrund der Erfindung

Aufgrund des in Zukunft immer wichtiger werdenden Lebenszyklus-Managements von Sterilgütern, steigt die Anforderung einer Überwachung und Rückverfolgbarkeit dieser Sterilgüter, insbesondere von medizintechnischen/ chirurgischen Instrumenten in einem Sterilisationskreislauf. Aus diesem Grund werden medizinische Instrumente aller Art mit Transpondern, insbesondere passive Transponder der RFID- (radiofrequency indentification) bzw. NFC- (near field communication) Technologie ausgestattet. Dadurch kann stets der Standort der medizinischen Instrumente identifiziert werden und eine Aussage darüber getroffen werden, ob diese den gesamten Wiederaufbereitungsprozess bzw. alle Wartungsschritte (korrekt) durchlaufen haben. Die Ausführung des Transponders in der Bauform des Glass-Tags ist dabei besonders vorteilhaft.

### Stand der Technik

Aus dem Stand der Technik sind bereits Vorrichtungen zum Anbringen von Transpondern an medizinische Instrumente bekannt. Beispielsweise wird in der Druckschrift EP 2 087 850 ein Transponder mithilfe eines Gehäuses an das medizinische Instrument, beispielsweise an dessen Griffstück, befestigt, wobei das Gehäuse einen Hohlraum zur Aufnahme des Transponders aufweist. Dabei kann das Gehäuse aus zwei getrennten Teilen bestehen, welche den Transponder sowie das Griffstück des Instruments in einer Aussparung bei einem Befestigungsvorgang umschließen und sich dann formschlüssig verbinden lassen. Eine weitere Befestigungsmöglichkeit besteht darin, den Transponder in ein über ein Scharnier klappbares Schutzgehäuse aufzunehmen, welches dann über einen flexiblen Riemen / Gurt an das medizinische Instrument geschnallt wird.

Aus der Druckschrift EP 3 146 477 ist ein metallisches Befestigungselement für metallische RFID-Tags bekannt. Das Befestigungselement wird über eine flache Grundfläche an einem medizinischen Instrument angebracht/ geklebt und nimmt den Transponder über eine durch Seitenflächen umschlossene Aussparung auf. Dabei sind der Transponder und das Befestigungselement elektrisch leitend miteinander verbunden, wodurch die Signalstärke bzw. Reichweite des Transponders, insbesondere bei der Befestigung an kleineren Objekten, verbessert werden kann.

WO 2014/045265 A1 offenbart einen weiteren Stand der Technik.

Ein Anbringen des Transponders nach den vorstehend genannten Möglichkeiten beeinflusst, aufgrund der Befestigung des Transponders an der Außenseite eines medizinischen Objekts und der dadurch entstehenden Hervorhebungen, das Handling sowie die Ergonomie der nachgerüsteten Objekte und kann außerdem eine medizinische Aufbereitung bzw. Reinigung erschweren.

Allgemein sind auch Möglichkeiten bekannt, einen Transponder durch Kleben/ Vergießen/ Schweißen oder in zuvor angebrachte Bohrungen an bzw. in ein medizinisches Instrumentengehäuse anzubringen. Allerdings sind diese Lösungen aufwendig und erfordern entsprechende Vorbereitungen, wie ein Sicherstellen einer sauberen und fettfreien Oberfläche der medizinischen Instrumente. Zudem ist ein Einpressen über Kraftschluss oder ein Kleben des Transponders aufgrund eines Setzens von Kunststoff nicht dauerhaft beständig.

Für ein Unterbringen eines Transponders, beispielsweise ein durch ein Gehäuse geschützter Glass-Tag, an einem medizinischen Produkt stehen oftmals nur kleine Wandstärken zur Verfügung. Damit sind klassische Schnappelemente wie Schnapphaken aufgrund deren notwendigen Dimensionen kaum realisierbar. Außerdem sind bestimmte Instrumente wie motorisch angetriebene Sägen während des Betriebes hohen Schwingungen ausgesetzt, sodass diese Schnappelemente in kleinen Baugrößen zu filigran sind und auf Dauer brechen können. Auch sogenannte Ringschnappverbindungen, die über eine Wulst in ein Instrumentengehäuse einrasten, haben den Nachteil, dass sie beim Montieren bzw. Einrasten durch eine Deformierung des Gehäuses mit dem darin aufgenommenen Transponder eine Kraft ausüben, die den Transponder schon während der Montage zerstören kann.

### Kurzbeschreibung der Erfindung

Daher liegt der vorliegenden Offenbarung die Aufgabe zugrunde, die vorstehend beschriebenen Nachteile zu vermeiden oder wenigstens abzumildern. Dabei soll ein Transponder, insbesondere ein passiver RFID-Tag der Glass-Tag Ausführung, einfach, sicher und platzsparend in/an einem medizinischen Produkt/ Instrument untergebracht/befestigt werden, wobei während dem Montagevorgang keine oder nur geringe Kräfte direkt auf den Glass-Tag wirken sollen. Des Weiteren sollen die medizinischen Instrumente für die erforderliche Nachrüstung auf einfache Art nachbearbeitet werden können. Ebenfalls soll durch diese Einbauart eine ausreichende Zuverlässigkeit und Empfangbarkeit des Transponders sichergestellt werden.

Diese Aufgabe wird offenbarungsgemäß durch ein Schutzgehäuse für einen oder eines Transponder(s) mit den Merkmalen des Patentanspruchs 1 und durch ein medizinisches System mit den Merkmalen des Anspruchs 6 gelöst. Vorteilhafte Ausgestaltungen und Weiterbildungen des Schutzgehäuses sind Gegenstand der Unteransprüche.

### Die Offenbarung betrifft demzufolge ein Schutzgehäuse zum

Unterbringen/Befestigen eines darin aufnehmbaren Transponders in/an einem medizinischen Produkt, insbesondere in/an einem chirurgischen Instrument und/ oder Medizinprodukt vorzugsweise in einem Sterilisationskreislauf. Dabei weist das Schutzgehäuse an mindestens zwei gegenüberliegenden Seiten mindestens zwei integrierte, sich vorzugsweise diagonal gegenüberliegende (d.h. in Gehäuse-Längsrichtung zueinander versetzte) Rastnasen/-leisten/-vorsprünge auf, die ausgebildet sind, um in Einkerbungen/ Befestigungskerben/Ausnehmungen innerhalb des medizinischen Produkts hinterschneidend einzurasten und das Schutzgehäuse weist Aussparungen jeweils gegenüberliegend der Rastnasen auf, wodurch das Schutzgehäuse während der Montage in seiner Längsachse tordierbar oder auslenkbar ist.

In anderen Worten kann ein vorzugsweise einstückig ausgebildetes Schutzgehäuse aus einem signaldurchlässigen Material, beispielsweise aus (flexiblem) Kunststoff, einen Transponder, insbesondere einen RFID-Tag in der Ausführung eines Glass-Tags, aufnehmen und diesen mithilfe von mindestens zwei Rastnasen/ Hervorhebungen/ Vorsprüngen, welche an wenigstens zwei gegenüberliegenden Seiten des Schutzgehäuses angeordnet sind und sich dabei diagonal gegenüberliegen, in ein medizinisches Produkt bzw. in einen Hohlraum des medizinischen Produkts unterbringen. Dabei weist das medizinische Produkt Einkerbungen/ Befestigungskerben/ Halterungen auf, in die die Rastnasen des Schutzgehäuses eingreifen. Eine Befestigung des Schutzgehäuses erfolgt dabei durch ein hinterschneidendes Einrasten der Rastnasen in die Einkerbungen am medizinischen Produkt. Vorzugsweise ist das Schutzgehäuse des Transponders dabei plan/ eben/ abschließend mit der Oberfläche des medizinischen Produkts.

Vorzugsweise ist das medizinische Produkt eine medizinische Vorrichtung oder ein medizinisches Instrument, ein monopolares/ bipolares HF-Instrument, eine chirurgische Klammer, eine chirurgische Zange, ein Behälter, eine chirurgische Schere ein Skalpell und/ oder dergleichen. Insbesondere kann das medizinische Produkt ein motorisch betriebenes Instrument mit einem Handstück sein, wobei der Motor in diesem integriert sein kann und/ oder ein Werkzeug, das mit dem Handstück in Koppeleingriff bringbar ist

Demnach wird sichergestellt, dass ein Transponder sicher und platzsparend in/an einem medizinischen Produkt aufgenommen werden kann, da die Ausführung des Schutzgehäuses keine filigran wegstehenden Elemente aufweist. Durch diese Befestigungsart kann auf einen aufwendigen Vorbereitungsprozess der Oberfläche des medizinischen Produkts verzichtet werden, welche beispielsweise bei einer Befestigung des Transponders durch Kleben oder Schweißen notwendig wäre. Ein weiterer Vorteil besteht darin, dass die Einkerbungen am Instrumentengehäuse relativ einfach und kostengünstig durch eine Nachbearbeitung oder schon während dem Herstellungsprozess angebracht werden können.

Gemäß einer vorteilhaften Ausführungsform sind die Rastnasen des Schutzgehäuses so ausgebildet, dass sie in die Einkerbungen innerhalb einer an dem Gehäuse des medizinischen Produkts ausgebildeten Nut hinterschneidend einrasten.

Anders ausgedrückt, weist das Gehäuse des medizinischen Produkts eine Nut oder Ausnehmung auf, in die das Schutzgehäuse (mit dem Transponder) eingelassen wird. Insbesondere kann die Form sowie die Tiefe der Nut derart an die Maße des Schutzgehäuses angepasst sein, dass das Schutzgehäuse im angebrachten / montierten Zustand komplett von der Nut umschlossen wird und dessen Oberfläche nahtlos an die des medizinischen Instruments anschließt, bzw. sogar (geringfügig) zurückversetzt ist. Entscheidend hierbei ist, dass das Schutzgehäuse bevorzugt nicht über die Oberfläche des Medizinprodukts hinausragt. Dabei sind die Einkerbungen, welche die Rastnasen des Schutzgehäuses aufnehmen, in der Nut des medizinischen Produkts angeordnet bzw. ausgebildet.

Dadurch wird sichergestellt, dass das Schutzgehäuse mit dem aufgenommenen Transponder gänzlich in dem Instrumentengehäuse untergebracht werden kann, womit einem Abstehen bzw. Herausstehen des Schutzgehäuses von der Oberfläche des medizinischen Produkts entgegengewirkt wird. Dies führt zu einem besseren Handling, vereinfacht den Reinigungsvorgang sowie das Lagern des medizinischen Produkts. Gleichzeitig ist diese Ausführung mit einer Nut am Gehäuse des medizinischen Produkts ebenfalls durch eine relativ einfache Nachbearbeitung, bzw. einem einfachen Herstellungsprozess möglich.

Das Schutzgehäuse weist (zusätzlich auch) Aussparungen jeweils gegenüberliegend der Rastnasen auf, d.h. jeweils einer Rastnase an einer Gehäuseseite ist eine Aussparung an der hiervon abgewandten Gehäuseseite zugeordnet.

Wenn daher an beiden (voneinander abgewandten Gehäuseseiten) jeweils Rastnasen angeordnet sind, so weisen die beiden (voneinander abgewandten Gehäuseseiten) jeweils auch Aussparungen in gleicher Anzahl auf, die jeweils einer der Rastnasen gegenüberliegen (beispielsweise abwechselnd oder nur einmalig). Auf diese Weise kann erreicht werden, dass das Schutzgehäuse während der Montage, d.h. bei einem Einsetzen der gehäuseseitigen Rastnase als ersten Montageschritt, gefolgt von einem Einfügen der gehäuseseitigen Aussparung als zweiter Montageschritt, in seiner Längsachse bzw. Torsionsachse tordieren bzw. sich auslenken kann. Die Rastnasen / Rastelemente des Schutzgehäuses weichen durch eine derartige Torsionsbewegung elastisch nach innen aus, das heißt in eine Richtung entgegengesetzt zum Gehäuse des medizinischen Produkts. Sobald alle Rastnasen in die jeweils vorgesehenen Einkerbungen am Gehäuse des medizinischen Produkts eingefügt worden sind bzw. in diese eingeschnappt / eingerastet sind, ist das Schutzgehäuse hinterschneidend am Gehäuse des medizinischen Produkts befestigt.

In anderen Worten ausgedrückt, befinden sich Aussparungen an den Seitenflächen des Schutzgehäuses, an denen (auch) die Rastnasen ausgebildet sind. Dabei liegt jeweils eine der Aussparungen direkt gegenüber einer der Rastnasen, die sich auf der entgegenliegenden Seite befindet. Beim Montieren des Schutzgehäuses können die Rastnasen ungehindert von den Einkerbungen am Gehäuse des medizinischen Produkts aufgenommen werden, da das Schutzgehäuse durch die Aussparung an der gegenüberliegenden Seite um einen kleinen Abstand in diese entweichen kann bis die Rastnase schließlich in die Einkerbung einrastet und das Schutzgehäuse wieder um den entwichenen Abstand zurückschnappt. Dadurch, dass das Schutzgehäuse mindestens zwei diagonal gegenüberliegende Rastnasen aufweist, kann das Schutzgehäuse bei der Montage an das medizinische Produkt in seiner Längsachse/ Hauptachse als tordierendes Schnappelement dienen.

Gemäß einem weiteren vorteilhaften Aspekt der Offenbarung sind die Rastnasen (an zwei sich gegenüberliegenden Seiten des Schutzgehäuses) entlang der Längsachse des Schutzgehäuses gegeneinander versetzt angeordnet. In anderen Worten, sind die mindestens zwei Rastnasen an den zwei gegenüberliegenden Seiten des Schutzgehäuses zueinander asymmetrisch bzw. abwechselnd angeordnet, sodass beim Einschnappen / Einrasten der Rastnasen in die entsprechenden Auskerbungen am medizinischen Produkt eine Torsionskraft entsteht.

Ferner, kann das Schutzgehäuse während der Montage durch Aussparungen innerhalb der Nut, die jeweils gegenüberliegend der Rastnasen ausgebildet sind, in seiner Längsachse tordieren bzw. sich auslenken. Das heißt, die Aussparungen befinden sich in dieser Ausführungsform nicht direkt an dem Schutzgehäuse des Transponders, sondern sind innerhalb der Nut am Gehäuse des medizinischen Produkts ausgebildet bzw. angeordnet. Die Wirkungsweise der Aussparungen in dieser Ausführungsform gleicht den Aussparungen, die direkt am Schutzgehäuse ausgebildet sind.

Aufgrund diesem tordierenden Montageprinzip des Schutzgehäuses besteht keine Gefahr, dass sich das Schutzgehäuse durch Kräfte, welche durch Vibrationen/ Schwingungen während dem Betriebszustand des medizinischen Produkts entstehen, insbesondere eines motorisch betriebenen medizinischen Instruments, von selbst aus den Einkerbungen im medizinischen Produkt loslöst bzw. herauswindet.

Das Schutzgehäuse kann den Transponder ferner in einer Bohrung entlang der Längsachse des Schutzgehäuses aufnehmen. Beispielsweise befindet sich der Transponder zentral im Schutzgehäuse und wird dabei der Länge nach vollständig von dem Schutzgehäuse umgeben.

Somit kann am gesamten Umfang des Transponders ein ausreichender Schutz vor den Kräften während der Montage des Schutzgehäuses bzw. während dem Betrieb des medizinischen Produkts sichergestellt werden. Außerdem erfordert die Aufnahmemöglichkeit, in Form einer Bohrung, einen geringen Herstellungsaufwand des Schutzgehäuses.

Die Offenbarung betrifft weiterhin ein Transpondersystem mit einem Schutzgehäuse und einem darin aufgenommenen Transponder, wobei der Transponder zusätzlich zu dem Schutzgehäuse von einer flexiblen Schutzschicht umgeben ist, die sich somit zwischen dem Transponder und dem Schutzgehäuse befindet.

**Im** montierten Zustand dient die Schutzschicht als Dämpfungseinheit zwischen dem Transponder und dem Schutzgehäuse. Hierbei wird der Transponder, bevor dieser in das Schutzgehäuse aufgenommen wird, zusätzlich mit einer flexiblen Schutzschicht, beispielsweise einer Silikonschicht, vollständig umgeben. Dabei weist der Transponder inklusive Schutzschicht etwas größere Maße auf als die Aufnahme in dem Schutzgehäuse, insbesondere in der Form einer Bohrung, wodurch das flexible Material der Schutzschicht während dem Aufnahmevorgang des Transponders in das Schutzgehäuse zusammengedrückt wird. Dementsprechend wird ein eventuelles Spiel zwischen dem Transponder und dem Schutzgehäuse verhindert.

Eine bevorzugte Ausführung mit einer zusätzlichen Schutzschicht als Dämpfung zwischen dem Transponder und dem Schutzgehäuse bietet eine weitere Möglichkeit die Sicherheit des Transponders vor extremen Vibrationen und Schwingungen im Betriebszustand zu erhöhen.

Die Ausführung eines Schutzgehäuses, welche sich wieder von dem medizinischen Produkt trennen lässt, ermöglicht ein Auswechseln bzw. Ersetzen von medizinischen Instrumenten, ohne dass der angebrachte / montierte Transponder inklusive Schutzgehäuse ausgetauscht werden muss. In bestimmten Fällen kann das Herauslösen der Transponder auch die Möglichkeit einer erneuten Zuweisung schaffen, da das Schutzgehäuse samt Transponder in ein medizinisches Objekt eines anderen Typs eingesetzt werden kann.

Weiter bevorzugt, weist das Schutzgehäuse ein Metallgehäuse zusätzlich zu dem Schutzgehäuse auf.

In anderen Worten ausgedrückt, wird das Schutzgehäuse mit einem zusätzlichen Metallgehäuse in das medizinische Produkt integriert. Dies ist vor allem dann notwendig, wenn das medizinische Produkt ein signaldurchlässiges Gehäuse aus Kunststoff aufweist. In diesem Fall fungiert das zusätzliche Metallgehäuse als ein Signalreflektor für die Signale des Transponders. Durch diese Signalverstärkung kann eine ausreichende Empfangbarkeit und Zuverlässigkeit gewährleistet werden.

Die Offenbarung betrifft weiterhin ein medizinisches System mit einem medizinischen Produkt und einem Schutzgehäuse. Die Rastnasen des Schutzgehäuses rasten in Einkerbungen innerhalb einer an einem Gehäuse des medizinischen Produkts ausgebildeten Nut hinterschneidend ein und das Schutzgehäuse ist während der Montage durch Aussparungen innerhalb der Nut, die jeweils gegenüberliegend der Rastnasen ausgebildet sind, und/oder durch Aussparungen am Schutzgehäuse jeweils gegenüberliegend der Rastnasen in seiner Längsachse tordierbar oder auslenkbar.

In einer bevorzugten Ausführungsform sind die Rastnasen so ausgebildet sind, dass das Schutzgehäuse nach der Montage von dem medizinischen Produkt unlösbar ist.

In einer weiteren alternativen, bevorzugten Ausführungsform sind die Rastnasen so ausgebildet sind, dass das Schutzgehäuse nach der Montage von dem medizinischen Produkt lösbar ist.

Mit anderen Worten ausgedrückt, können die Rastnasen des Schutzgehäuses einerseits so ausgebildet sein, dass sich diese nach dem Verankern in die im medizinischen Schutzgehäuse vorgesehenen Einkerbungen nicht mehr loszulösen sind. Andererseits kann die Form der Rastnasen auch so ausgebildet sein, dass das Schutzgehäuse nach der Montage durch eine Betätigung seitens des Benutzers wieder von dem medizinischen Produkt getrennt werden kann.

Demnach ist das medizinische System derart ausgebildet, dass das Schutzgehäuse während der Montage durch Aussparungen innerhalb der Nut des medizinischen Produkts, welche jeweils gegenüberliegend der Rastnasen ausgebildet sind, in seiner Längsachse tordierbar oder auslenkbar ist. Anders ausgedrückt, sind die Aussparungen, welche jeweils gegenüber einer Rastnase angeordnet sind, nicht direkt am Schutzgehäuse ausgebildet, sondern innerhalb der Nut des medizinischen Produkts. Auch eine derartige Anordnung der Aussparungen ermöglicht ein Tordieren des Schutzgehäuses während der Montage, sodass die Rastnasen elastisch nach innen ausweichen können und so in die entsprechenden Einkerbungen am medizinischen Produkt einrastbar sind.

Vorzugsweise, weist die Nut des medizinischen Systems zusätzliche Materialausnehmungen aufweist, sodass im montierten Zustand des Schutzgehäuses von der Unterseite des Schutzgehäuses bzw. von innen zugängliche Hohlräume zwischen dem Schutzgehäuse und dem medizinischen Produkt entstehen bzw. ausgebildet sind.

Mit anderen Worten ausgedrückt, weist das Gehäuse des medizinischen Produkts/Objekts an der an diesem ausgebildeten Nut, vorzugsweise an der Grundfläche der Nut, zusätzliche Materialausnehmungen auf, welche sich insbesondere an den Seiten entlang der Längsachse des Schutzgehäuses befinden. Diese Materialausnehmungen bilden im montierten Zustand des Schutzgehäuses vorteilhafte Hohlräume/ Lücken zwischen den zwei Komponenten.

Aufgrund dieser von der Unterseite des Schutzgehäuses bzw. von innen zugänglichen Hohlräume können sich Partikel oder andere ungewollte Substanzen schwer zwischen dem Schutzgehäuse und dem medizinischen Produkt ablagern. Gleichzeitig sind die Hohlräume für Reinigungsvorgänge leicht / einfach zugänglich, wodurch stets eine reinigungsgerechte medizinische Aufbereitung der medizinischen Instrumente sichergestellt werden kann.

### Kurzbeschreibung der Figuren

Der offenbarte Gegenstand wird nun anhand vorteilhafter Ausführungsformen und unter Bezugnahme auf die dazugehörenden Figuren näher erläutert. Dabei sind die Figuren lediglich schematischer Natur und dienen ausschließlich dem Verständnis der Erfindung. Es wird darauf hingewiesen, dass die Merkmale der einzelnen Ausführungsformen untereinander ausgetauscht werden sowie in einer beliebigen Kombination auftreten können.
Fig. 1 ist eine Vorderansicht des erfindungsgemäßen Schutzgehäuses für einen Transponder.
Fig. 2 ist eine Seitenansicht des erfindungsgemäßen Schutzgehäuses für einen Transponder.
Fig. 3 ist eine Darstellung zur Veranschaulichung der Dimensionen eines Glass-Tag Transponders
Fig. 4 ist eine Vorderansicht des erfindungsgemäßen Schutzgehäuses mit einem darin aufgenommenen Glass-Tag.
Fig. 5 ist eine Darstellung der Nut am Gehäuse eines medizinischen Instruments zur Aufnahme des erfindungsgemäßen Schutzgehäuses.
Fig. 6 ist eine Darstellung der Nut am Gehäuse eines medizinischen Instruments mit einem darin untergebrachten Schutzgehäuse.
Fig. 7 ist eine Schnittansicht durch das medizinische Instrument und dem montierten Schutzgehäuse mit Glass-Tag.
Fig. 8 ist eine Schnittansicht des Schutzgehäuses während des Montagevorgangs an einem medizinischen Instrument mit den dabei auftretenden Kräften und Momenten.
Fig. 9 ist eine Draufsicht auf das Schutzgehäuse während des Montagevorgangs an einem medizinischen Instrument mit den dabei auftretenden Kräften und Momenten.
Fig. 10 ist eine Schnittansicht entlang der Längsachse des Schutzgehäuses und dem darin aufgenommenen Glass-Tag mit einer zusätzlichen Schutzschicht.
Fig. 11 ist eine Schnittansicht des Schutzgehäuses mit aufgenommenen Glass-Tag in einem medizinischen Instrument mit zusätzlichen Materialausnehmungen.
Fig. 12 ist eine weitere Schnittansicht des Schutzgehäuses mit aufgenommenen Glass-Tag in einem medizinischen Instrument mit zusätzlichen Materialausnehmungen.

### Figurenbeschreibung

Nachfolgend wird die vorliegende Offenbarung sowie die vorteilhaften Ausführungsformen anhand der Figuren beschrieben.

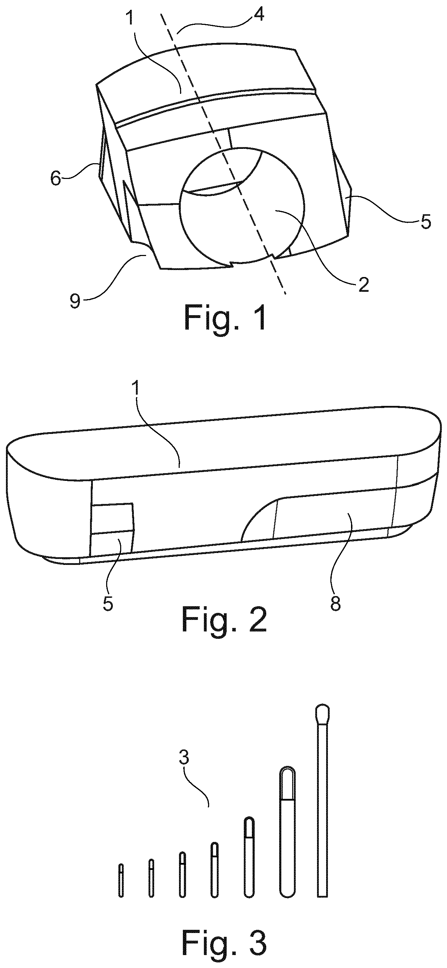

Figur 1 ist eine Darstellung zur Veranschaulichung des erfindungsgemäßen Schutzgehäuses 1 mit einer Längsachse 4. Das Schutzgehäuse 1 ist dabei länglich ausgebildet und weist entlang der Längsachse 4 eine kreisförmige (Durchgangs-)Bohrung 2 zur Aufnahme eines Transponders 3 auf. Erfindungsgemäß sind jeweils an zwei voneinander abgewandten, im Wesentlichen parallel zueinander verlaufenden Seitenflächen des Schutzgehäuses 1, welche auch parallel entlang der Bohrung 2 verlaufen, Rastnasen 5, 6 ausgebildet, die sich von der jeweiligen Seitenfläche nach außen erstrecken. Eine erste Rastnase 5 ist dabei an einem Längs-Endabschnitt des Schutzgehäuses 1 an der einen Seitenfläche ausgebildet, während eine zweite Rastnase 6 an dem anderen Längs-Endabschnitt an der anderen, abgewandten Seitenfläche ausgebildet ist, und zwar derart, dass sich die zwei Rastnasen 5, 6 schräg (bezüglich des Gehäuses diagonal) gegenüberliegen. Zusätzlich weist das Schutzgehäuse 1 an den beiden voneinander abgewandten Seitenflächen auch Aussparungen 8, 9 auf, welche jeweils gegenüber den Rastnasen 5, 6 der jeweils anderen Seitenfläche angeordnet sind.

Figur 2 ist eine weitere Darstellung des Schutzgehäuses 1 aus einer Seitenansicht. Die jeweiligen Enden (Endabschnitte) des Schutzgehäuses 1 sind konvex abgerundet. Die in Fig. 2 dargestellte eine seitliche Rastnase 5 befindet sich hier an dem linken Ende (Endabschnitt) des Schutzgehäuses 1, wobei sich die längliche Aussparung 8 an dem rechten Ende (Endabschnitt) befindet. Dabei ist die Aussparung 8 so ausgebildet, dass sich diese entlang der Längsachse 4 des Schutzgehäuses 1 von einem Ende bis in etwa zu der Mitte durchzieht, an welcher Stelle die Aussparung 8 kurvenförmig in die ursprüngliche Seitenfläche übergeht. Die Aussparung 9 (siehe Fig. 1) ist an der gegenüberliegenden Seitenfläche des Schutzgehäuses 1 ausgebildet und liegt dabei diagonal gegenüber der Aussparung 8.

Figur 3 ist eine Darstellung zur Veranschaulichung von erfindungsgemäß verwendbaren Transpondern. Die Transponder können insbesondere als passive RFID-Transponder ausgebildet sein, wobei eine vorteilhafte Ausführung die eines Glass-Tags 3 ist. Die Ausführung des Glass-Tags 3 besitzt eine pillenartige Form und weist eine kreisförmige Querschnittsfläche sowie abgerundete Enden auf. **In** der Figur 3 werden unterschiedliche Größen des Glass-Tags 3 einem handelsüblichen Streichholz gegenübergestellt, um die Größenordnung der Transponder zu verdeutlichen. Derartige Glass-Tags 3 zählen im Wesentlichen zum Stand der Technik, weshalb keine nähere Beschreibung notwendig ist.

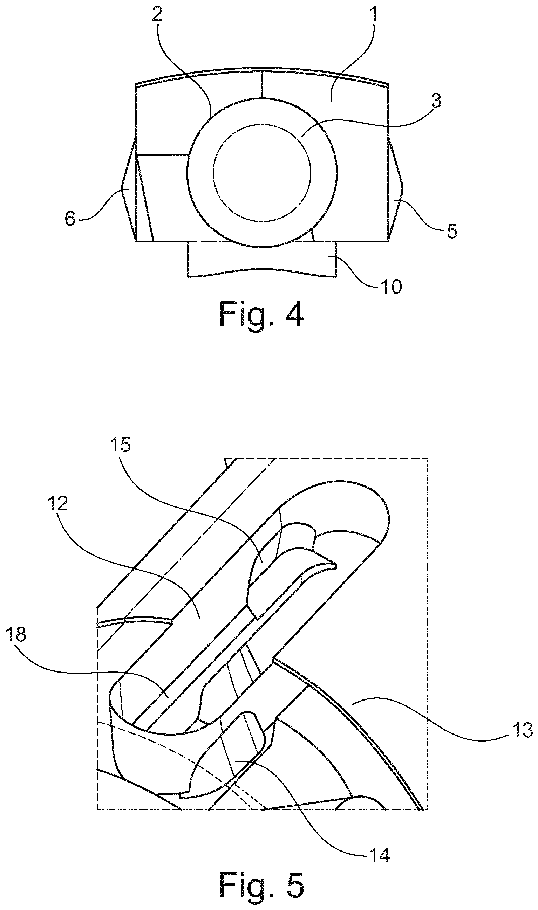

Figur 4 ist eine Vorderansicht des erfindungsgemäßen Schutzgehäuses 1 mit einem darin aufgenommenen Transponder in der Ausführung eines Glass-Tags 3. Die Bohrung 2 entlang der Längsachse 4 des Schutzgehäuses 1 weist einen dem Glass-Tag 3 angepassten Durchmesser auf, sodass dieser über den Umfang vollständig und passgenau von der Bohrung 2 aufgenommen werden kann. Außerdem ist die Länge des Schutzgehäuses 1 an die Länge des Glass-Tags 3 so angepasst, dass die Enden des Glass-Tags 3 nicht aus dem Schutzgehäuse 1 herausragen. Ferner, weist die Grundfläche des Schutzgehäuses 1 einen zusätzlichen quaderförmigen Abschnitt 10 auf, welcher hervorsteht und dabei an der Unterseite abgerundet ist. Die sich jeweils diagonal gegenüberliegenden Rastnasen 5, 6 an den Seitenflächen des Schutzgehäuses 1 weisen dreiecksförmige Querschnittsflächen auf, die achssymmetrisch bezüglich einer horizontalen Mittellinie sind.

Figur 5 zeigt eine Nut 12, welche an der Oberfläche bzw. Gehäuse eines medizinischen Instruments 13 ausgebildet / angeordnet ist und welche an die Form des erfindungsgemäßen Schutzgehäuses 1 (mit einem darin aufgenommenen Glass-Tag 3) angepasst ist. Hierbei kann diese Nut 12 während dem Herstellungsprozesses des Instruments 13 oder aber auch in einem zusätzlichen Nachbearbeitungsvorgang der Oberfläche, beispielsweise durch einen Fräsvorgang, angebracht werden. In dieser vorteilhaften Ausführungsform hat die Nut 12 eine sogenannte Stadionform, das heißt eine Form, die aus einem Rechteck mit zwei anliegenden Halbkreisen ausgebildet ist. Seitlich innerhalb der Nut 12 sind Einkerbungen 14, 15 ausgebildet, deren Positionen jeweils an die Rastnasen 5, 6 des erfindungsgemäßen Schutzgehäuses 1 angepasst sind. Somit liegen sich diese Einkerbungen 14, 15 innerhalb der Nut 12 ebenfalls diagonal gegenüber. Des Weiteren sind längliche Vorsprünge 17, 18 entlang der zwei Längsseiten der Nut 12 ausgebildet, an die das Schutzgehäuse 1 im montierten Zustand aufliegen kann.

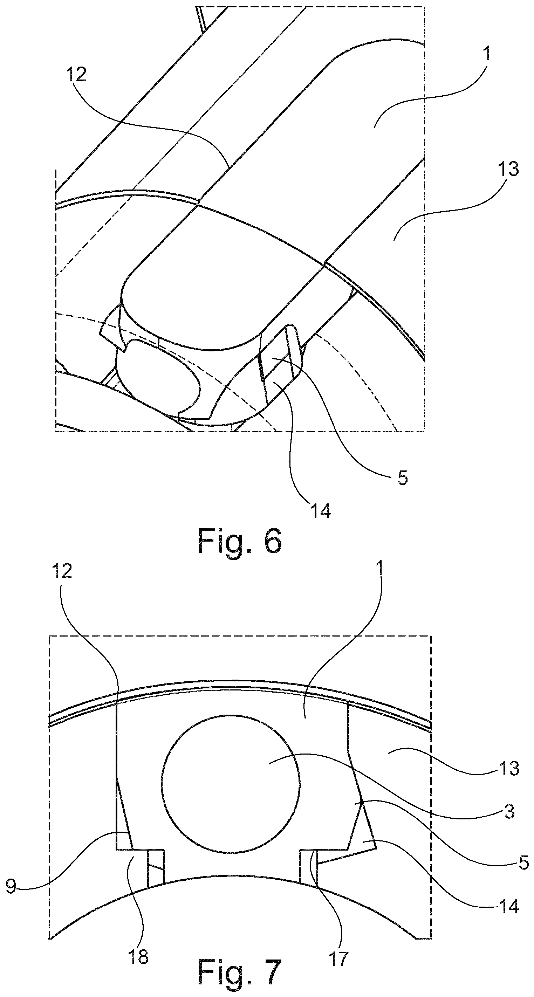

Figur 6 ist eine Darstellung zur Veranschaulichung des Schutzgehäuses 1 (mit dem darin aufgenommenen Glass-Tag 3) in einem montierten Zustand. Dabei ist das Schutzgehäuse 1 innerhalb der Nut 12 am/im medizinischen Instrument 13 aufgenommen, wobei die Tiefe der Nut 12 an die Höhe des erfindungsgemäßen Schutzgehäuses 1 so angepasst ist, dass sich die Oberseite des Schutzgehäuses 1 auf derselben Ebene der Instrumentenoberfläche befindet oder (geringfügig) zurückversetzt ist, weswegen die Oberfläche des Instruments 13 nicht beeinträchtigt wird. In dieser vorteilhaften Ausführung ist die Größe der Nut 12 an das Schutzgehäuse 1 angepasst, sodass im montierten Zustand kein Spiel zwischen dem Schutzgehäuse 1 und dem Gehäuse des medizinischen Instruments 13 vorliegt. In dieser Ansicht ist die Rastnase von der Einkerbung 14 aufgenommen und befindet sich im eingerasteten Zustand.

Figur 7 zeigt eine Schnittansicht senkrecht zur Längsachse 4 des Schutzgehäuses 1, die durch die Nut 12 im Instrumentengehäuse mit dem darin untergebrachten erfindungsgemäßen Schutzgehäuse 1 verläuft. Das Schutzgehäuse 1 weist dabei einen Glass-Tag 3 in dessen Bohrung 2 auf. Die Schnittebene verläuft auf der Höhe einer Rastnase 5 und der gegenüberliegenden Aussparung 9 am Schutzgehäuse 1 sowie der zugehörigen Einkerbung 14 in der Nut des medizinischen Instruments 13, das heißt durch die vordere Hälfte des Schutzgehäuses 1. Der Querschnitt der Einkerbung 14 in der Nut 12 zur Aufnahme der Rastnase 5 des Schutzgehäuses 1 ist so ausgebildet, dass die obere Hälfte der Rastnase 5 an der Einkerbung 14 anliegt und hinterschneidend aufgenommen wird. Gleichzeitig liegt ein Spiel zwischen dem medizinischen Instrument 13 und der unteren Hälfte der Rastnase 5 vor, und zwar sowohl an der Seite als auch an der Unterseite der Rastnase 5, welche der Grundfläche des Schutzgehäuses 1 entspricht. Aufgrund der Aussparung 9 und dem zuvor beschriebenen Spiel zwischen Rastnase 5 und dem medizinischen Instrument 13, kann diese Hälfte des Schutzgehäuses 1 während dem Montagevorgang bezüglich einer Achse, die senkrecht aus der Schnittebene herausragt, (im Uhrzeigersinn gemäß der Fig. 7) tordieren. Erst durch diese tordierende Bewegung kann die erfindungsgemäße Rastnase 5 des Schutzgehäuses 1 von der Einkerbung 14 innerhalb der Nut 12 hinterschneidend aufgenommen werden. Die Aussparung 8, Einkerbung 15 und Rastnase 6 in der zweiten Querschnitts-Ebene, welche durch die hintere Hälfte des Schutzgehäuses 1 verläuft, besitzen dieselbe Funktion wie ihre jeweils diagonal gegenüberliegende Gegenstücke, wodurch die hintere Hälfte des Schutzgehäuses 1 entgegen dem Uhrzeigersinn tordieren kann. Die Grundfläche des Schutzgehäuses 1 liegt abgesehen von den Stellen, an denen die Rastnasen 5, 6 ausgebildet sind, an Vorsprüngen 17, 18 des Instrumentengehäuses an. Durch diese Vorsprünge 17, 18 und den hinterschneidenden Eingriff der Rastnasen 5, 6 in die Einkerbungen 14, 15 ist das Schutzgehäuse 1 in der Vertikalen fixiert.

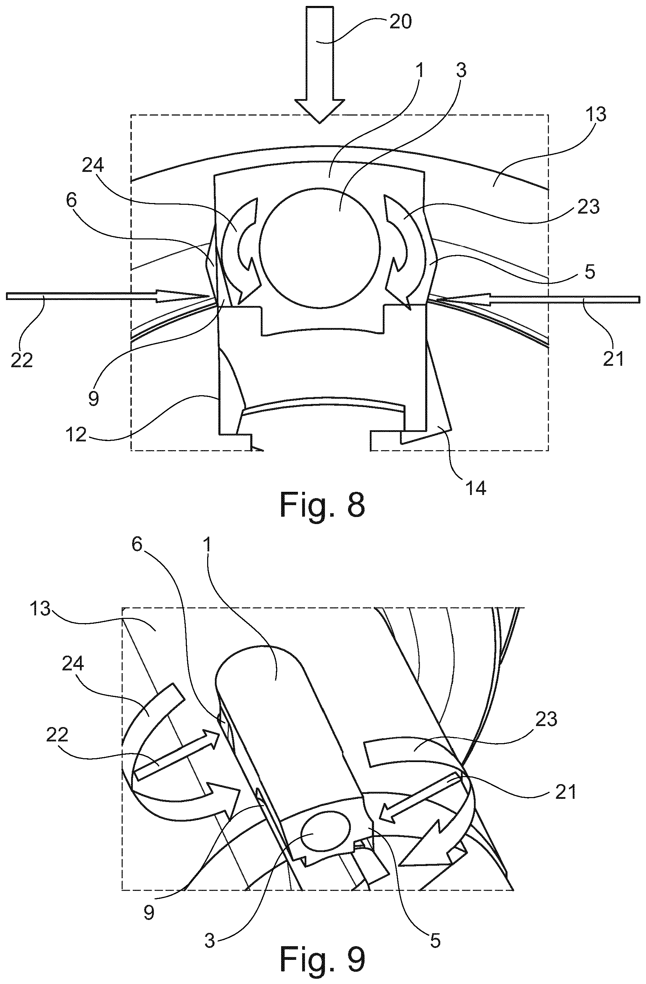

Figuren 8 und 9 zeigen jeweils das durch die Ebene der vorderen Rastnase 5 geschnittene Schutzgehäuse 1 mit einem darin aufgenommenen Glass-Tag 3 während einem Montagevorgang in das medizinische Instrument 13. Zusätzlich sind die Kräfte 20, 21, 22 und Momente 23, 24 eingezeichnet, die während des Montagevorgangs auf das Schutzgehäuse 1 wirken. Dabei stellt Figur 8 eine Schnittansicht und Figur 9 eine Draufsicht dar. Zum Unterbringen bzw. Einrasten des Schutzgehäuses 1 in der im medizinischen Instrument 13 vorgesehenen Nut 12, wirkt eine Montagekraft 20 vertikal auf das Schutzgehäuse 1, womit das Schutzgehäuse 1 inklusive Glass-Tag 3 in die Nut 12 des medizinischen Instruments 13 gedrückt wird. Während diesem Vorgang wirken durch das Instrumentengehäuse an den Seiten des Schutzgehäuses 1 jeweils horizontale Rastkräfte 21, 22 auf die vordere und hintere Rastnase 5, 6. Ein Torsionsmoment 23 (gemäß der Fig. 8 und 9 im Uhrzeigersinn) an der vorderen Hälfte des Schutzgehäuses 1 und ein Gegen-Torsionsmoment 24 (gegen den Uhrzeigersinn) in der hinteren Hälfte des Schutzgehäuses 1 stellen die Bewegungsmöglichkeiten während dem Einrastvorgang dar, welche sich dank der vorstehend beschriebenen Aussparungen 8, 9 und Form der Einkerbungen 14, 15 in der Nut 12 ergeben.

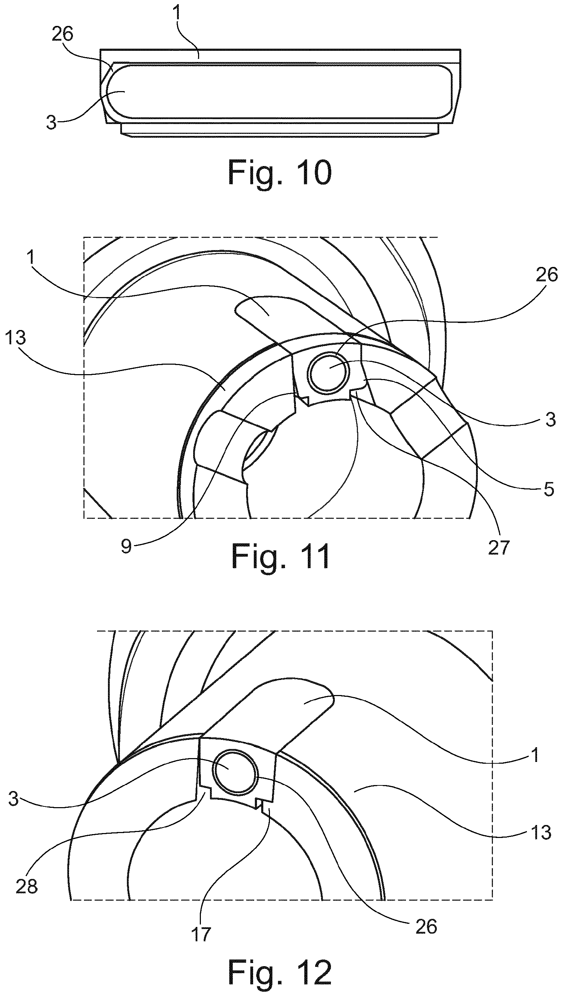

Figur 10 ist eine Schnittansicht entlang der Längsachse 4 des erfindungsgemäßen Schutzgehäuses 1 mit einem Glass-Tag 3, welcher vollständig in der (Durchgangs-)Bohrung 2 untergebracht ist. Der gesamte Glass-Tag 3 ist dabei von einer flexiblen Schutzschicht 26 umschlossen, um diesen von erhöhten Vibrationen und Schwingungen zu schützen. Die Dicke der Schutzschicht 26 ist dabei so gewählt, dass die Schutzschicht 26 beim Einführen des Glass-Tags 3 in die Bohrung 2 des Schutzgehäuses 1 zusammengedrückt wird. Somit besteht im montierten Zustand des Glass-Tags 3 kein Spiel zwischen diesem und dem umgebenden Schutzgehäuse 1, wodurch ein ungewolltes Herauslösen/ Durchrutschen verhindert wird.

Figuren 11 und 12 zeigen eine Schnittansicht einer weiteren vorteilhaften Ausführungsform des medizinischen Instruments 13 mit einem untergebrachten Glass-Tag 3 in einem Schutzgehäuse 1. Die seitlichen Vorsprünge 17, 18 des in der Nut 12 des medizinischen Instruments 13, die zur vertikalen Fixierung des Schutzgehäuses 1 dienen, sind dabei nicht durchgehend ausgebildet und weisen teilweise Materialausnehmungen 27, 28 auf. Vorzugsweise sind diese unterhalb der Rastnasen 5, 6 und der Aussparungen 8, 9 des Schutzgehäuses 1 ausgebildet. Dadurch ergeben sich von innen / vom Inneren (des medizinischen Produkts) zugängliche Hohlräume, welche eine leichtere Reinigung im eingebauten Zustand des Schutzgehäuses 1 begünstigen. Gleichzeitig kann die vertikale Fixierung durch die Vorsprünge 17, 18 an den restlichen Abschnitten der Unterseite des Schutzgehäuses 1 sichergestellt werden. Diese vorteilhafte Ausführungsform kann dabei auch eine Schutzschicht 26 zwischen dem Glass-Tag 3 und dem Schutzgehäuse 1 aufweisen oder auch nicht.

### Bezugszeichenliste

- 1: Schutzgehäuse
- 2: (Durchgangs-)Bohrung
- 3: Transponder/ Glass-Tag
- 4: Längsachse des Schutzgehäuses
- 5: Rastnase
- 6: Rastnase
- 8: Aussparung
- 9: Aussparung
- 10: quaderförmiger Abschnitt
- 12: Nut
- 13: medizinisches Instrument/ medizinisches Produkt
- 14: Einkerbung
- 15: Einkerbung
- 17: Vorsprung
- 18: Vorsprung
- 20: Montagekraft
- 21: Rastkraft
- 22: Rastkraft
- 23: Torsionsmoment
- 24: Torsionsmoment
- 26: Schutzschicht
- 27: Materialausnehmung
- 28: Materialausnehmung

## Patentansprüche

1. Schutzgehäuse (1) zum Unterbringen eines darin aufnehmbaren Transponders (3) in einem medizinischen Produkt (13), insbesondere in einem chirurgischen Instrument und/oder Medizinprodukt in einem Sterilisationskreislauf, **dadurch gekennzeichnet, dass**
das Schutzgehäuse (1) an zwei gegenüberliegenden Seiten mindestens zwei integrierte sich diagonal gegenüberliegende Rastnasen (5, 6) aufweist, die ausgebildet sind, um in Einkerbungen (14, 15) innerhalb des medizinischen Produkts (13) hinterschneidend einzurasten, und das Schutzgehäuse (1) Aussparungen (8, 9) jeweils gegenüberliegend der Rastnasen (5, 6) aufweist, wodurch das Schutzgehäuse (1) während der Montage in seiner Längsachse (4) tordierbar oder auslenkbar ist.

2. Schutzgehäuse (1) nach Anspruch 1, **dadurch gekennzeichnet, dass** die Rastnasen (5, 6) entlang der Längsachse (4) des Schutzgehäuses (1) gegeneinander versetzt angeordnet sind.

3. Schutzgehäuse nach Anspruch 1 oder 2, **dadurch gekennzeichnet, dass** das Schutzgehäuse (1) den Transponder (3) in einer Bohrung (2) entlang der Längsachse (4) des Schutzgehäuses (1) aufnimmt.

4. Transpondersystem mit einem Schutzgehäuse (1), vorzugsweise nach einem der Ansprüche 1 bis 3, und einem darin aufgenommenen Transponder (3), **dadurch gekennzeichnet, dass** der Transponder (3) zusätzlich zu dem Schutzgehäuse (1) von einer flexiblen Schutzschicht (26) umgeben ist, die sich somit zwischen dem Transponder (3) und dem Schutzgehäuse (1) befindet.

5. Schutzgehäuse (1) nach einem der Ansprüche 1 bis 3, **dadurch gekennzeichnet, dass** das Schutzgehäuse (1) ein Metallgehäuse zusätzlich zu dem Schutzgehäuse (1) aufweist.

6. Medizinisches System mit einem medizinischen Produkt (13), und einem Schutzgehäuse (1), vorzugsweise nach einem der Ansprüche 1 bis 3, **dadurch gekennzeichnet, dass** Rastnasen (5, 6) des Schutzgehäuses (1) in Einkerbungen (14, 15) innerhalb einer an einem Gehäuse des medizinischen Produkts (13) ausgebildeten Nut (12) hinterschneidend einrasten und das Schutzgehäuse (1) während der Montage durch Aussparungen innerhalb der Nut (12), die jeweils gegenüberliegend der Rastnasen (5, 6) ausgebildet sind, und/oder durch Aussparungen (8, 9) am Schutzgehäuse (1) jeweils gegenüberliegend der Rastnasen (5, 6) in seiner Längsachse (4) tordierbar oder auslenkbar ist.

7. Medizinisches System nach Anspruch 6, **dadurch gekennzeichnet, dass** die Nut (12) zusätzliche Materialausnehmungen (27, 28) aufweist, die so ausgebildet sind, dass im montierten Zustand des Schutzgehäuses (1) von dessen Unterseite zugängliche Hohlräume zwischen dem Schutzgehäuse (1) und dem medizinischen Produkt (13) entstehen.

8. Medizinisches System nach Anspruch 6 oder 7, **dadurch gekennzeichnet, dass** die Rastnasen (5, 6) so ausgebildet sind, dass das Schutzgehäuse (1) nach der Montage von dem medizinischen Produkt (13) unlösbar ist.

9. Medizinisches System nach Anspruch 6 oder 7, **dadurch gekennzeichnet, dass** die Rastnasen (5, 6) so ausgebildet sind, dass das Schutzgehäuse (1) nach der Montage von dem medizinischen Produkt (13) lösbar ist.

## Claims

1. A protective housing (1) for accommodating a transponder (3) receivable therein in a medical device (13), in particular in a surgical instrument and / or medical device in a sterilization circuit, **characterized in that**
the protective housing (1) on two opposite sides has at least two integrated snap-in noses (5, 6) diagonally opposite to each other formed so as to engage in notches (14, 15) within the medical device (13) in an undercutting manner, and the protective housing (1) has recesses (8, 9) respectively opposite the snap-in noses (5, 6), whereby the protective housing (1) is twistable or deflectable along its longitudinal axis (4) during assembly.

2. The protective housing (1) according to claim 1, **characterized in that** the snap-in noses (5, 6) are offset from one another along the longitudinal axis (4) of the protective housing (1).

3. The protective housing according to claim 1 or 2, **characterized in that** the protective housing (1) receives the transponder (3) in a hole (2) along the longitudinal axis (4) of the protective housing (1).

4. A transponder system with a protective housing (1), preferably according to any of claims 1 to 3, and a transponder (3) accommodated therein, **characterized in that** the transponder (3) additionally to the protective housing (1) is surrounded by a flexible protective layer (26), which is thus located between the transponder (3) and the protective housing (1).

5. The protective housing (1) according to any of claims 1 to 3, **characterized in that** the protective housing (1) has a metal housing in addition to the protective housing (1).

6. A medical system with a medical device (13), and a protective housing (1), preferably according to any of claims 1 to 3, **characterized in that** snap-in noses (5, 6) of the protective housing (1) snap in notches (14, 15) within a groove (12) formed on the housing of the medical device (13) in an undercutting manner and the protective housing (1) during assembly is twistable or deflectable along its longitudinal axis (4) by way of recesses within the groove (12), respectively formed opposite to the snap-in noses (5, 6), and/or by way of recesses (8, 9) on the protective housing (1), respectively opposite the snap-in noses (5, 6).

7. The medical system according to claim 6, **characterized in that** the groove (12) has additional material recesses (27, 28) formed in such a way that in the mounted state of the protective housing (1), cavities that are accessible from its bottom side, are formed between the protective housing (1) and the medical device (13).

8. The medical system according to claim 6 or 7, **characterized in that** the snap-in noses (5, 6) are configured so that the protective housing (1) after the assembly is not detachable from the medical device (13).

9. The medical system according to claim 6 or 7, **characterized in that** the snap-in noses (5, 6) are configured so that the protective housing (1) after the assembly is detachable from the medical device (13).

## Revendications

1. Boîtier de protection (1) pour le logement d'un transpondeur (3) pouvant être reçu en son sein dans un produit médical (13), en particulier dans un instrument chirurgical et/ou un produit médical dans un cycle de stérilisation, **caractérisé en ce que**
le boîtier de protection (1) présente, au niveau de deux côtés opposés, au moins deux nez d'encliquetage intégrés (5, 6) diagonalement opposés, qui sont conçus pour s'encliqueter en contre-dépouille dans des encoches (14, 15) à l'intérieur du produit médical (13), et le boîtier de protection (1) présente des évidements (8, 9) situés respectivement à l'opposé des nez d'encliquetage (5, 6), moyennant quoi le boîtier de protection (1) peut être tordu ou dévié sur son axe longitudinal (4) pendant le montage.

2. Boîtier de protection (1) selon la revendication 1, **caractérisé en ce que** les nez d'encliquetage (5, 6) sont disposés de manière décalée les uns par rapport aux autres le long de l'axe longitudinal (4) du boîtier de protection (1).

3. Boîtier de protection selon la revendication 1 ou 2, **caractérisé en ce que** le boîtier de protection (1) reçoit le transpondeur (3) dans un alésage (2) s'étendant le long de l'axe longitudinal (4) du boîtier de protection (1).

4. Système de transpondeur avec un boîtier de protection (1), de préférence selon l'une quelconque des revendications 1 à 3, et un transpondeur (3) reçu en son sein, **caractérisé en ce que** le transpondeur (3) est entouré, en plus du boîtier de protection (1), d'une couche de protection souple (26) qui se trouve ainsi entre le transpondeur (3) et le boîtier de protection (1).

5. Boîtier de protection (1) selon l'une quelconque des revendications 1 à 3, **caractérisé en ce que** le boîtier de protection (1) présente un boîtier métallique en plus du boîtier de protection (1).

6. Système médical avec un produit médical (13) et un boîtier de protection (1), de préférence selon l'une quelconque des revendications 1 à 3, **caractérisé en ce que** des nez d'encliquetage (5, 6) du boîtier de protection (1) s'encliquettent en contre-dépouille dans des encoches (14, 15) à l'intérieur d'une rainure (12) formée au niveau d'un boîtier du produit médical (13), et le boîtier de protection (1) peut, pendant le montage, être tordu ou dévié sur son axe longitudinal (4) via des évidements à l'intérieur de la rainure (12), évidements qui sont formés respectivement à l'opposé des nez d'encliquetage (5, 6) et/ou via des évidements (8, 9) au niveau du boîtier de protection (1) respectivement à l'opposé des nez d'encliquetage (5, 6).

7. Système médical selon la revendication 6, **caractérisé en ce que** la rainure (12) présente des évidements de matériau supplémentaires (27, 28) qui sont conçus de sorte que, à l'état monté du boîtier de protection (1), des cavités accessibles depuis son côté inférieur sont créées entre le boîtier de protection (1) et le produit médical (13).

8. Système médical selon la revendication 6 ou 7, **caractérisé en ce que** les nez d'encliquetage (5, 6) sont conçus de sorte que le boîtier de protection (1) soit indétachable du produit médical (13) après le montage.

9. Système médical selon la revendication 6 ou 7, **caractérisé en ce que** les nez d'encliquetage (5, 6) sont conçus de sorte que le boîtier de protection (1) soit détachable du produit médical (13) après le montage.
